# EUROPÄISCHE PATENTSCHRIFT

(11) **EP 1 530 492 B1**
(45) Veröffentlichungstag und Bekanntmachung des Hinweises auf die Patenterteilung: **24.06.2009**
(21) Anmeldenummer: 03797209.8
(22) Anmeldetag: 24.07.2003
(51) Int. Cl.: A61L 29/08, A61L 29/14, A61L 31/10, A61L 31/14, G03F 7/028

(54) **VERFAHREN ZUR IMMOBILISIERUNG VON HYDROGEL-BILDENDEN POLYMEREN AUF POLYMERSUBSTRATOBERFLAECHEN**
METHOD FOR IMMOBILIZING HYDROGEL-BONDING POLYMERS ON POLYMER SUBSTRATE SURFACES
PROCEDE POUR IMMOBILISER DES POLYMERES FORMANT UN HYDROGEL SUR DES SURFACES DE SUBSTRAT POLYMERE

(30) Priorität: 22.08.2002 DE 10238559
(43) Veröffentlichungstag der Anmeldung: 18.05.2005
(73) Patentinhaber: Fresenius Medical Care Deutschland GmbH, 61352 Bad Homburg v.d.H. (DE)
(72) Erfinder: DRÖSCHEL, Stefan, 66125 Saarbrücken (DE); FISLAGE, Rainer, 66606 St. Wendel (DE)
(74) Vertreter: Müller-Boré & Partner Patentanwälte
(86) Internationale Anmeldenummer: PCT/EP2003/008180
(87) Internationale Veröffentlichungsnummer: WO 2004/026356

(56) Entgegenhaltungen:
- WO-A-03/055611
- US-A- 4 766 160
- US-A1- 2001 024 697
- US-B1- 6 410 645

## Beschreibung

Die vorliegende Erfindung betrifft ein Verfahren zur Immobilisierung eines Polymers auf Polyvinylpyrrolidon (PVP)-Basis auf einer entsprechenden Polymersubstratoberfläche. Insbesondere findet die vorliegende Erfindung im Bereich der Medizintechnik Anwendung, beispielsweise zur Herstellung einer Beschichtung von blutkontaktierenden Oberflächen, wie bei der Hämodialyse, sowie einer Beschichtung von Harnkathetern, Venenkathetern, Stents und anderen Oberflächen.

Hydrogelbeschichtungen werden zur Verbesserung der Biokompatibilität von Oberflächen eingesetzt, die in Kontakt mit Körperflüssigkeiten, insbesondere Blut, gebracht werden. Derartige Hydrogelbeschichtungen sollen dabei die Belegung der Oberflächen mit Zellen und Proteinen vermindern, die zur Aktivierung körpereigener Abwehrsysteme gegen Fremdmaterialien führen bzw. die Blutgerinnung fördern. Darüber hinaus werden derartige Hydrogelbeschichtungen zur Erhöhung der Gleitfähigkeit und Verbesserung der Benetzbarkeit für wässrige Lösungen auf das entsprechende Substrat aufgebracht.

Bekannte Beispiele für die dazu verwendeten Hydrogele sind Polyethylenglykol (PEG) sowie Polyvinylpyrrolidon (PVP), die bereits bei der Herstellung entsprechender medizintechnischer Produkte in die Matrix der jeweiligen Basispolymeren eingelagert werden. Wenn die dabei verwendeten Basispolymere in geschmolzener oder gelöster Form mit den Hydrogelverbindungen nicht mischbar sind, können die Substratoberflächen nachträglich damit beschichtet werden.

Zur kovalenten Fixierung derartiger Hydrogele an das zu behandelnde Substrat sind verschiedene Verfahren bekannt. So wird beispielsweise kurzwellige elektromagnetische Strahlung im γ-Strahlungs- bzw. Röntgenbereich eingesetzt, die eine Fixierung der Hydrogele auch auf chemisch wenig reaktiven Oberflächen ermöglicht. Dabei werden häufig reaktive Zwischenprodukte in der Tiefe der verwendeten Basispolymere erzeugt. Ferner können die Farbe und die mechanische Stabilität des Produktes nachteilig verändert werden.

Bei rein chemischen Immobilisierungsverfahren werden im allgemeinen wenig reaktive Substratmaterialien durch aggressive Reagenzien wie z.B. Chlorsulfonsäure aktiviert, um anschließend Liganden auf der zu beschichtenden Oberfläche zu binden. Dabei ergeben sich insbesondere produktionstechnische Probleme mit aggressiven bzw. toxischen Reagenzien. Gerade im Fall von Polyvinylpyrrolidon erweist es sich jedoch als schwierig, ein entsprechendes Hydrogel auf Polymersubstratoberflächen wie z.B. Polypropylen (PP) oder Polyvinylchlorid (PVC) zu binden.

In US-A-6,159,645 wird ein Verfahren zur Quervernetzung von Polyvinylpyrrolidon bei der Herstellung von Bildröhren in der Elektroindustrie beschrieben, worin das Natriumsalz der 4,4'-Diazidostyrol-2,2'-disulfonsäure als Photoinitiator eingesetzt wird. Dieses Natriumsalz ist jedoch aus toxikologischen Gründen für die Herstellung medizintechnischer Produkte bedenklich.

Vitamin B2 (Riboflavin) ist bereits seit langem als Photoinitiator für die Polymerisation von reaktiven Monomeren wie Acrylamid oder Vinylpyrrolidon bekannt (vgl. US-A-2,850,445). Nach UV-Bestrahlung der in homogener Lösung vorliegenden Monomer/Initiator-Gemische werden die entsprechenden Hydrogele gebildet, die insbesondere bei einer Quervernetzung präzipitieren. Im vorstehenden US-Patent wird dabei die Bildung von Hydrogelen aus den Monomervorstufen vorgesehen. Besonders im Bereich der biochemischen Analytik ist der Polymerisationsstart mit Vitamin B2 als Photoinitiator bekannt. Hier werden die dadurch erzeugten Polyacrylamidgele als Matrix für die elektrophoretische Trennung von Proteinen verwendet. Bei besonders empfindlichen Proteinen wird das gebräuchlichere Ammoniumperoxodisulfat (APS)/N,N,N',N'-Tetramethylethylendiamin (TEMED)-Initiatorsystem durch Riboflavin ersetzt, da dieses als besonders schonend bekannt ist. So kann beispielsweise auch der Einschluß von lebenden Zellen in einer Hydrogelmatrix mit Riboflavin als Photoinitiator erreicht werden; vgl. US-A-6,224,893. Bei diesem Verfahren werden Gemische aus reaktiven Monomeren und Polymeren verwendet, die nach der Reaktion ein interpenetrierendes Netzwerk bilden, das die Zellen einschließt.

US 2002/0122872 A1 beschreibt ein Verfahren zur Beschichtung einer Materialoberfläche unter Verwendung von beispielsweise Thioxanthon als Photoinitiator.

US-A-4,766,160 beschreibt die Herstellung einer photopolymerisierbaren Zusammensetzung unter Verwendung eines entsprechenden Photoinitiators. US-B1-6,410,645 beschreibt ein Verfahren, worin auf ein Gewebe *in vivo* eine wässrige Lösung eines Gel-bildenden Makromonomers, welches spezifisch dadurch gekennzeichnet ist, dass es mindestens kovalent verknüpfte Polymerblöcke umfasst, aufgebracht wird. US 2001/024697 beschreibt eine durch Pfropfpolymerisation von Makromeren mit Oberflächenphotoinitiierung in der Gegenwart eines Benzophenons gebildete Oberflächenbeschichtung. WO 03/055611 A1 beschreibt ein Verfahren zur Beschichtung von Stents, Kathetern bzw. Implantaten unter Verwendung eines Gemisches, umfassend N-Vinylpyrollidon als Monomerbaustein und Thioxanthon als Photoinitiator.

Der vorliegenden Erfindung liegt somit die Aufgabe zugrunde, ein einfaches, kostengünstiges und weitgehend schonendes Verfahren zur Immobilisierung von PVP-Polymeren auf Polymeroberflächen bereitzustellen. Ein derartiges Verfahren soll keine nachteiligen Veränderungen der Polymeroberflächen hervorrufen und keine toxikologisch bedenklichen Initiatoren verwenden, welche die Arbeitssicherheit beim Produktionsprozeß beeinträchtigen oder als Verunreinigung in der erzeugten Hydrogelschicht verbleiben.

Diese Aufgabe wird durch die in den Ansprüchen gekennzeichneten Ausführungsformen gelöst.

Insbesondere wird ein Verfahren zur Immobilisierung eines Polymers auf PVP-Basis auf einer Polymersubstratoberfläche bereitgestellt, wobei eine Zusammensetzung, umfassend mindestens ein Polymer und PVP-Basis mit einem K-Wert von 15 bis 120 und mindestens eine nicht-toxische Photoinitiatorverbindung und ein oder mehrere Lösungsmittel mindestens bereichsweise auf die Oberfläche eines Polymersubstrats aufgebracht wird und anschließend nach Trocknen einer Behandlung mit elektromagnetischer Strahlung unterworfen wird, so daß das Polymer auf PVP-Basis auf der Polymersubstratoberfläche darauf immobilisiert wird. Die vorliegende Erfindung stellt somit die Immobilisierung bzw. Fixierung eines bereits fertigen PVP-Hydrogels an polymere Oberflächen, im Gegensatz zu dem im Stand der Technik an sich üblichen Verfahren der Herstellung eines Hydrogels aus entsprechenden Monomeren, bereit. Erfindungsgemäß erfolgt die Immobilisierung des Hydrogels somit, nachdem die polymere Oberfläche bereits physikalisch mit dem Hydrogel beschichtet worden ist.

Die zur Aktivierung eingesetzte elektromagnetische Strahlung unterliegt keiner Beschränkung. Grundsätzlich können Wellenlängen verwendet werden, die zur Anregung des Systems aus Initiator/Hydrogel-bildendes Polymer/Oberflächenpolymer geeignet sind. In einer bevorzugten Ausführungsform des erfindungsgemäßen Verfahrens wird zur Immobilisierung des PVP-Polymers nach Aufbringen und Trocknen der Hydrogel-bildenden Zusammensetzung elektromagnetische Strahlung im ultravioletten bis sichtbaren Bereich des Spektrums, vorzugsweise im Bereich von 170 nm bis 600 nm, eingesetzt. Alternativ kann im Rahmen der vorliegenden Erfindung auch Strahlung vergleichbarer Energie, die durch Zwei-Photonenanregung erzeugt werden kann, beispielsweise Laserstrahlung, eingesetzt werden.

Das Polymer auf Polyvinylpyrrolidon (PVP)-Basis schließt Polyvinylpyrrolidon, Polyvinylpyrrolidon enthaltende Copolymere und Derivate des Polyvinylpyrrolidons bzw. deren Copolymere ein. Das Polymer auf Polyvinylpyrrolidon-Basis weist einen K-Wert im Bereich von 15 bis 120, besonders bevorzugt einen K-Wert bei 120, auf. Der K-Wert ist ein Maß für das Molekulargewicht (H. Fikentscher, Cellulose-Chemie 13 (1932), Seiten 58 bis 64 und 71 bis 74). Der K-Wert der Polymeren kann über Startermenge, Temperatur und pH-Wert im Rahmen der Polymerisationsbedingungen des in der vorliegenden Erfindung einzusetzenden PVP-Polymers eingestellt werden. Erfindungsgemäß ist es auch möglich, Polyvinylpyrrolidon enthaltende Copolymere einzusetzen. Es ist dabei bevorzugt, daß wenigstens 50 %, mehr bevorzugt 75 %, der Monomereinheiten in derartigen Copolymeren Vinylpyrrolidon-Monomer sind. Als Comonomere können beispielhaft Vinylacetat und Vinylether angeführt werden. Als Derivate des Polyvinylpyrrolidons können beispielsweise solche Derivate eingesetzt werden, die befähigt sind, physiologische Reaktionen zu verändern oder zu modulieren. Dies schließt insbesondere Verbindungen ein, welche die Blutgerinnung oder Funktionen des Immunsystems beeinflussen können. Als solche Polyvinylpyrrolidonderivate können beispielsweise Oligosaccharid-substituierte PVP-Polymere und Peptid-substituierte PVP-Polymere angeführt werden. Ein Beispiel hierfür sind PVP-Heparin-Komplexe, wie beispielsweise in US-A-4,239,664 beschrieben, auf die hier explizit Bezug genommen wird. Des weiteren kommen als solche Polyvinylpyrrolidonderivate Alkyl-substituierte Polymere, beispielsweise (C₁-C₆)-Alkyl-substituierte Derivate des Polyvinylpyrrolidons, in Frage. Die Alkylsubstitution kann dabei sowohl am Alkylenpolymergrundgerüst als auch am Pyrrolidonring vorgesehen sein.

Als Polymersubstrat können jegliche, insbesondere in der Medizintechnik üblicherweise verwendeten Polymere bzw. Copolymere verwendet werden, um erfindungsgemäß mit einer PVP-Hydrogelschicht versehen zu werden. Vorzugsweise können als Polymersubstrat, auf welches erfindungsgemäß eine PVP-Hydrogelschicht aufgebracht wird, Polymermaterialien, ausgewählt aus Polyethylen, Polypropylen, Polyvinylchlorid, Polycarbonat oder Polyurethan oder Gemischen bzw. Copolymeren davon, eingesetzt werden. Des weiteren können beispielsweise hierfür auch SEBS-Polymersubstrate verwendet werden. Das zu beschichtende Polymersubstrat kann dabei ein Dialysator, Schlauch, Katheter, Stent oder Urether sein oder mindestens einen Teil davon bilden.

Die in der Hydrogel-bildenden Zusammensetzung eingesetzten Konzentrationen an Hydrogel-bildendem PVP-Polymer und Initiatorverbindung unterliegen ebenfalls keiner spezifischen Beschränkung. Die Konzentrationen sollten jedoch hoch genug sein, um die beschriebenen Obertlächeneigenschaften zu gewährleisten und eine nicht auswaschbare Hydrogelschicht zu bilden. Die Viskosität der Hydrogel-bildenden Zusammensetzung sollte für das anzuwendende Beschichtsverfahren entsprechend geeignet sein. Die genau einzusetzende Konzentration kann dabei von einem Fachmann entsprechend gewählt werden. Beispielsweise wird bei Verwendung einer 1 Gew.-% bis 10 Gew.-%igen Lösung von Polyvinylpyrrolidon (K-Wert von 120) in Ethanol oder Dimethylacetamid die Initiatorverbindung üblicherweise in einer Menge von 0,1 bis 0,5 Gew.-%, bezogen auf die Hydrogel-bildende Zusammensetzung, verwendet. Die genaue Konzentration hängt dabei beispielsweise von der Löslichkeit des Initiators und des Hydrogel-bildenden PVP-Polymers im jeweils verwendeten Lösungsmittel ab.

Die Hydrogel-bildende Zusammensetzung umfasst neben dem mindestens einen Hydrogel-bildenden PVP-Polymer und der mindestens einen Initiatorverbindung ein oder mehrere Lösungsmittel. Die Wahl des Lösungsmittels unterliegt dabei keiner Beschränkung. Beispielhaft können hier Methanol, Ethanol, Dimethylacetamid, Acetonitril, etc., bzw. deren Gemische angeführt werden. Initiatorverbindung und Hydrogel-bildendes PVP-Polymer sollten dabei im gleichen Lösungsmittel bzw. Lösungsmittelsystem löslich sein, um eine homogene Verteilung von Hydrogel-bildendem PVP-Polymer und Initiator in der getrockneten Reaktandenschicht zu gewährleisten.

Im Rahmen des erfindungsgemäßen Verfahrens wird zur Immobilisierung des entsprechenden PVP-Polymers mindestens eine nicht-toxische Photoinitiatorverbindung eingesetzt. Unter einer nicht-toxischen bzw. physiologisch unbedenklichen Photoiniatorverbindung wird im Rahmen der vorliegenden Erfindung eine Verbindung mit einem LD50 (Ratte) von ≥ 500 mg/kg, günstiger ≥ 950 mg/kg und am günstigsten ≥ 2000 mg/kg verstanden. Vorzugsweise ist die nicht-toxische Photoinitiatorverbindung eine solche Verbindung, ausgewählt aus der Gruppe, bestehend aus Flavinen, Flavonen, Flavonoiden und deren Derivate sowie Nicotinsäureamid (Vitamin B3) und dessen Derivate und Thioxanthon. Als Flavin-Derivate können insbesondere die in N¹⁰-Position substituierten (C₁-C₆)Alkyl- und (C₁-C₆)Alkoxy-Derivate sowie Riboflavin angeführt werden. Als Flavon-Derivate können beispielsweise Rutin (Quercetin-3-rutinosid) und Morin (2',3,4',5,7-Pentahydroxyflavon) angeführt werden. Als Flavonoid-Derivate können beispielsweise Verbindungen aus der Gruppe der Flavonole, Flavanole, Flavanone, Anthocyane und Isoflavonoide angeführt werden, soweit sie physiologisch unbedenklich sind. Besonders bevorzugt wird im Rahmen der vorliegenden Erfindung Riboflavin, Rutin (Quercetin-3-rutinosid), Morin (2',3,4',5,7-Pentahydroxyflavone) und Nicotinsäureamid, am bevorzugtesten Nicotinsäureamid, eingesetzt.

Im Rahmen der vorliegenden Erfindung wurde überraschenderweise festgestellt, daß nicht-toxische Photoinitiatorverbindungen wie insbesondere solche, ausgewählt aus der Gruppe, bestehend aus Flavinen, Flavonen, Flavonoiden und deren Derivate sowie Nicotinsäure und deren Derivate und Thioxanthon, nicht nur als Photoinitiator zur Polymerisation von ungesättigten Monomeren in homogener Lösung geeignet sind, sondern auch als Photoinitiator zur Immobilisierung von bereits fertigen, polymeren Hydrogelen auf Polyvinylpyrrolidon-Basis auf entsprechenden Polymeroberflächen geeignet sind. Dazu wird eine Hydrogel-bildende Zusammensetzung von mindestens einem Hydrogel-bildenden PVP-Polymer und mindestens einer nicht-toxischen Photoinitiatorverbindung, wie insbesondere einer solchen, ausgewählt aus der Gruppe, bestehend aus Flavinen, Flavonen, Flavonoiden und deren Derivate sowie Nicotinsäure und deren Derivate und Thioxanthon, auf einer Polymeroberfläche mit elektromagnetischer Strahlung vorzugsweise im ultravioletten bis sichtbaren Bereich des Spektrums behandelt. Derart erzeugte, erfindungsgemäße Hydrogelbeschichtungen sind danach auf der Polymersubstratoberfläche immobilisiert, d.h. weder durch kochendes Wasser noch durch einen Autoklavierprozeß von der Polymeroberfläche zu entfernen. Ohne daran gebunden zu sein, deuten diese Eigenschaften möglicherweise auf eine kovalente Verknüpfung der Hydrogelschicht auf der Polymeroberfläche hin, so daß eine Freisetzung solcher Polymere gerade bei Blutkontakt minimiert wird. Die derart behandelte Polymersubstratoberfläche wird durch das erfindungsgemäße Aufbringen eines derartigen Hydrogels hydrophil, was beispielsweise an einer verringerten Oberflächenspannung bzw. höheren Benetzbarkeit durch Wasser erkennbar ist.

Bei Verwendung von insbesondere Riboflavin als Photoinitiator liegt ein besonderer Vorteil der vorliegenden Erfindung darin, daß die derart behandelte Polymersubstratoberfläche bei Bestrahlung mit ultraviolettem Licht eine Fluoreszenz zeigt, die besonders günstig zur Qualitätskontrolle der Hydrogelbeschichtung verwendet werden kann. Ohne den zugrunde liegenden Mechanismus theoretisch beschreiben zu wollen, kann das Vorliegen der Fluoreszenz darauf hinweisen, daß das erfindungsgemäß als Initiatorverbindung eingesetzte Riboflavin, dessen Fluoreszenzeigenschaften bekannt sind, in die Hydrogelschicht eingebaut wird.

Das gleichzeitige Vorliegen des Hydrogel-bildenden PVP-Polymers in der Hydrogelschicht kann bei der Verwendung von Polymeren auf PVP-Basis auch durch die Komplexbildungsfähigkeit des PVP mit verschiedenen Farbstoffen nachgewiesen werden. So bildet bekanntlich eine lod/lod-Kalium Lösung, die auch als Lugol'sches Reagenz bekannt ist, einen bräunlich gefärbten Komplex mit PVP. Das bekannte Dragendorff-Reagenz bildet einen Komplex von orangebrauner Farbe mit PVP. Beide Farbreaktionen sind auf beispielsweise PVP/Riboflavin beschichteten Oberflächen positiv. Wird eine Beschichtungszusammensetzung bzw. Hydrogel-bildende Zusammensetzung ohne eine entsprechende Photoinitiatorverbindung verwendet, so wird keine haftende Hydrogelschicht nach der UV-Bestrahlung erhalten. Wird die erfindungsgemäß eingesetzte Initiatorverbindung durch ein APS/TEMED Initiatorsystem ersetzt, so wird ebenfalls keine haftende Hydrogelschicht auf PVP-Basis erhalten.

Die vorliegende Erfindung wird unter Bezugnahme auf die folgenden Beispiele näher erläutert.

### Beispiele

### Beispiel 1

Riboflavin (Sigma, R-7649, EC NO 201-5071) wurde in absolutem Ethanol bis zur Sättigung bei Raumtemperatur gelöst und die nicht gelösten Anteile wurden abfiltriert. In dieser Lösung wurde PVP K120 (ISP) mit einem Massenanteil von 4% gelöst. Diese Beschichtungslösung wurde auf Polymeroberflächen aufgebracht und nach dem Trocknen mit UV-VIS-Licht der Wellenlänge 170 bis 600 nm (Gerät: Fusion 300, Lampe D-Strahler) für 1 bis 120 Sekunden bestrahlt. Der Abstand zur UV-VIS-Lampe betrug 100 mm. Die Oberflächen wurden dann für 60 Minuten in destilliertem Wasser gekocht oder für 40 Minuten mit Wasserdampf bei 121°C überströmt. Die Hydrogelschicht konnte dann durch ihre Fluoreszenz oder die Anfärbbarkeit mit lod/lod-Kalium bzw. mit dem Dragendorff-Reagenz nachgewiesen werden.

Es können auch andere Lösungsmittel wie beispielsweise Dimethylacetamid (DMAC) und deren Gemische eingesetzt werden.

### Beispiel 2

Eine wie in Beispiel 1 hergestellte Hydrogel-bildende Beschichtungslösung unter Verwendung von DMAC als Lösungsmittel wurde durch eine Stempelmaschine der Firma Tampoprint auf den Polyurethanverguß eines Hämodialysators aufgebracht und in der Wärme getrocknet. Nach der UV-VIS-Bestrahlung konnte eine PVP-Hydrogelschicht auf der Polymeroberfläche nachgewiesen werden, die autoklavierbar war, d.h. durch einen Autoklavierprozeß von der Polymersubstratoberfläche nicht zu entfernen war.

### Beispiel 3

Eine wie in Beispiel 1 hergestellte Hydrogel-bildende Beschichtungslösung unter Verwendung von Ethanol als Lösungsmittel wurde mittels eines Spincoating-Verfahrens gleichmäßig auf den Innenraum der Dialysatkappe (Bereich mit Blutkontakt) eines Hämodialysators verteilt, getrocknet und mit UV-VIS-Licht entsprechend bestrahlt. Das Material der Dialysatkappe war Polypropylen oder Polycarbonat, das gegebenenfalls vor dem Auftrag der Hydrogel-bildenden Lösung durch eine Coronabehandlung aktiviert worden war. Nach der UV-VIS-Bestrahlung konnte eine PVP-Hydrogelschicht auf der Polymeroberfläche nachgewiesen werden, die autoklavierbar war, d.h. durch einen Autoklavierprozeß bzw. Kochen mit Wasser von der Polymersubstratoberfläche nicht zu entfernen war.

### Beispiel 4

Morin (2',3,4',5,7-Pentahydroxyflavon) wurde bei Raumtemperatur bis zur Sättigung in absolutem Ethanol gelöst und die nicht gelösten Anteile abfiltriert. In dieser Lösung wurde PVP K120 (ISP) mit einem Masseanteil von 4% gelöst. Diese Beschichtungslösung wurde auf Polymeroberflächen aufgebracht und nach dem Trocknen mit UV-VIS-Licht der Wellenlänge 170 bis 600 nm (Gerät: Fusion 300, Lampe D-Strahler) für 1 bis 120 s bestrahlt. Der Abstand zur UV-VIS-Lampe betrug 100 mm. Die Oberflächen wurden dann für 60 Minuten in destilliertem Wasser gekocht oder für 40 Minuten mit Wasserdampf bei 121°C überströmt. Die resultierenden Oberflächen waren hydrophil und konnten mit dem Dragendorff-Reagenz angefärbt werden.

Es können auch andere Lösungsmittel wie beispielsweise Dimethylacetamid (DMAC) und deren Gemische eingesetzt werden.

### Beispiel 5

Rutin (Quercetin-3-rutinosid) wurde bei Raumtemperatur bis zur Sättigung in absolutem Ethanol gelöst und die nicht gelösten Anteile abfiltriert. In dieser Lösung wurde PVP K120 (ISP) mit einem Masseanteil von 4% gelöst. Diese Beschichtungslösung wurde auf Polymeroberflächen aufgebracht und nach dem Trocknen mit UV-VIS-Licht der Wellenlänge 170 bis 600 nm (Gerät: Fusion 300, Lampe D-Strahler) für 1 bis 120 s bestrahlt. Der Abstand zur UV-VIS-Lampe betrug 100 mm. Die Oberflächen wurden dann für 60 Minuten in destilliertem Wasser gekocht oder für 40 Minuten mit Wasserdampf bei 121°C überströmt. Die resultierenden Oberflächen waren hydrophil und konnten mit dem Dragendorff-Reagenz angefärbt werden.

Es können auch andere Lösungsmittel wie beispielsweise Dimethylacetamid (DMAC) und deren Gemische eingesetzt werden.

### Beispiel 6

Es wurde eine 4%-ige PVP K120 (ISP)/Isopropanol-Lösung hergestellt. Anschließend wurden 0,33 g Nicotinsäureamid in 100 g dieser Lösung gelöst. Diese Beschichtungslösung wurde auf Polymeroberflächen aufgebracht und nach dem Trocknen mit UV-VIS-Licht der Wellenlänge 170 bis 600 nm (Gerät: Fusion 300, Lampe D-Strahler) für 1 bis 60 s im Lampenfokus bestrahlt. Der Abstand zur UV-VIS-Lampe betrug 100 mm. Die Oberflächen wurden dann für 60 Minuten in destilliertem Wasser gekocht oder für 40 Minuten mit Wasserdampf bei 121°C überströmt. Die resultierenden Oberflächen waren hydrophil und konnten mit dem Dragendorff'-Reagenz angefärbt werden.

Es können auch andere Lösungsmittel wie beispielsweise Dimethylacetamid (DMAC) und deren Gemische eingesetzt werden.

Ferner wurde ein Schlauch aus Polyvinylchlorid bzw. Polypropylen mit der in Beispiel 6 hergestellten Hydrogel-bildenden Beschichtungslösung in Ethanol gefüllt und nach ihrer Entfernung in dünner Schicht getrocknet. Nach der UV-VIS-Bestrahlung konnte eine PVP-Hydrogelschicht auf der Polymeroberfläche nachgewiesen werden, die autoklavierbar war, d.h. durch einen Autoklavierprozeß bzw. Kochen mit Wasser für 1 h von der Schlauchinnenoberfläche nicht zu entfernen war.

Das erfindungsgemäße Verfahren ist dabei nicht auf Schläuche einer bestimmten Geometrie bzw. einer bestimmten Materialzusammensetzung beschränkt. Grundsätzlich kommt jedes Material in Frage, auf dem sich in der beschriebenen Weise eine Hydrogelschicht mit den beschriebenen Eigenschaften immobilisieren läßt. Voraussetzung ist die Zugänglichkeit der PVP-Polymer/Initiator-Reaktandenschicht für die eingesetzte Wellenlänge und die Erzielbarkeit einer hinreichend hohen Intensität der elektromagnetischen Strahlung. Neben der Hydrogelschicht im Inneren eines Schlauches kann selbstverständlich auch eine Hydrogelschicht auf der Außenseite eines Schlauches erzeugt werden.

### Beispiel 7

Es wurde eine 4%-ige PVP K120 (ISP)/Isopropanol-Lösung hergestellt. Anschließend wurden 0,33 g Thioxanthon in 100 g dieser Lösung gelöst. Diese Beschichtungslösung wurde auf Polymeroberflächen aufgebracht und nach dem Trocknen mit UV-VIS-Licht der Wellenlänge 170 bis 600 nm (Gerät: Fusion 300, Lampe D-Strahler) für 1 bis 60 s im Lampenfokus bestrahlt. Der Abstand zur UV-VIS-Lampe betrug 100 mm. Die Oberflächen wurden dann für 60 Minuten in destilliertem Wasser gekocht oder für 40 Minuten mit Wasserdampf bei 121°C überströmt. Die resultierenden Oberflächen waren hydrophil und konnten mit dem Dragendorff-Reagenz angefärbt werden.

Es können auch andere Lösungsmittel wie beispielsweise Dimethylacetamid (DMAC) und deren Gemische eingesetzt werden.

## Patentansprüche

1. Verfahren zur Immobilisierung eines Polymers auf Polyvinylpyrollidon (PVP)-Basis auf einer Polymersubstratoberfläche, wobei eine Zusammensetzung, umfassend mindestens ein Polymer auf PVP-Basis mit einem K-wert von 15 bis 120 und mindestens eine nichttoxische Photoinitiatorverbindung und ein oder mehrere Lösungsmittel, mindestens bereichsweise auf die Oberfläche eines Polymersubstrats aufgebracht wird und anschließend nach Trocknen einer Behandlung mit elektromagnetischer Strahlung unterworfen wird, so daß das Polymer auf PVP-Basis auf der Polymersubstratoberfläche darauf immobilisiert wird.

2. Verfahren nach Anspruch 1, wobei zur Immobilisierung elektromagnetische Strahlung im ultravioletten bis sichtbaren Bereich des Spektrums, vorzugsweise im Bereich von 170 m bis 600 nm, eingesetzt wird.

3. Verfahren nach Anspruch 1 oder 2, wobei das Polymer auf Polyvinylpyrrolidon-Basis Polyvinylpyrrolidon, Polyvinylpyrrolidon enthaltende Copolymere, Derivate des Polyvinylpyrrolidons sowie deren Copolymere einschließt.

4. Verfahren nach einem der Ansprüche 1 bis 3, wobei das Polymersubstrat aus einem Polymermaterial, ausgewählt aus Polyethylen, Polypropylen, Polyvinylchlorid, Polycarbonat, SEBS oder Polyurethan oder Gemischen davon, aufgebaut ist.

5. Verfahren nach einem der Ansprüche 1 bis 4, wobei das Polymersubstrat ein Dialysator, Schlauch, Katheter, Stent oder Urether ist oder mindestens einen Teil davon bildet.

6. Verfahren nach einem der Ansprüche 1 bis 5, wobei die nicht-toxische Photoinitiatorverbindung aus der Gruppe, bestehend aus Flavinen, Flavonen, Flavonoiden und deren Derivate sowie Nicotinsäureamid und dessen Derivate und Thioxanthon, ausgewählt ist.

7. Verfahren nach Anspruch 6, wobei die Initiatorverbindung Riboflavin, Morin, Rutin oder ein Gemisch davon ist.

8. Verfahren nach Anspruch 6, wobei die Initiatorverbindung Nicotinsäureamid ist.

9. Verfahren nach Anspruch 6, wobei die Initiatorverbindung Thioxanthon ist.

## Claims

1. A method of immobilizing a polyvinylpyrrolidone (PVP)-based polymer on a surface of a polymer substrate, wherein a composition comprising at least one PVP-based polymer having a K value of 15 to 120 and at least one non-toxic photoinitiator compound and one or more solvents, is applied to the surface of the polymer substrate at least in areas, and then, after drying, is subjected to treatment with electromagnetic radiation, so that the PVP-based polymer is immobilized on the surface of the polymer substrate.

2. The method according to claim 1, wherein electromagnetic radiation in the ultraviolet to visible range of the spectrum, preferably in the range from 170 m to 600 nm, is used for immobilization.

3. The method according to claim 1 or 2, wherein the polyvinylpyrrolidone-based polymer contains copolymers containing polyvinylpyrrolidone, derivatives of polyvinylpyrrolidone and their copolymers.

4. The method according to any one of claims 1 to 3, wherein the polymer substrate is made of a polymer material chosen from polyethylene, polypropylene, polyvinyl chloride, polycarbonate, SEBS or polyurethane or mixtures thereof.

5. The method according to any one of claims 1 to 4, wherein the polymer substrate is a dialyser, hose, catheter, stent or urinary catheter or at least part of one.

6. The method according to any one of claims 1 to 5, wherein the non-toxic photoinitiator compound is chosen from the group composed of flavins, flavones, flavonoids and their derivatives, as well as nicotinic acid amide and its derivatives and thioxanthone.

7. The method according to claim 6, wherein the initiator compound is riboflavin, morin, rutin or a mixture thereof.

8. The method according to claim 6, wherein the initiator compound is nicotinic acid amide.

9. The method according to claim 6, wherein the initiator compound is thioxanthone.

## Revendications

1. Procédé d'immobilisation d'un polymère à base de polyvinylpyrrolidone (PVP) sur une surface de substrat polymère, une composition comprenant au moins un polymère à base de PVP présentant une valeur K de 15 à 120 et au moins un composé photo-initiateur non toxique et un ou plusieurs solvants, étant appliquée au moins en partie sur la surface d'un substrat polymère et ensuite, étant soumise àprès séchage à un traitement par rayonnement électromagnétique de sorte que le polymère à base de PVP soit immobilisé sur celle-ci.

2. Procédé selon la revendication 1, dans lequel, pour l'immobilisation, on utilise un rayonnement électromagnétique dans la plage des ultraviolets à la lumière visible du spectre, de préférence dans une plage de 170 m à 600 nm.

3. Procédé selon la revendication 1 ou 2, dans lequel le polymère à base de polyvinylpyrrolidone comprend de la polyvinylpyrrolidone, des copolymères contenant de la polyvinylpyrrolidone, des dérivés de polyvinylpyrrolidone et leurs copolymères.

4. Procédé selon l'une des revendications 1 à 3, dans lequel le substrat polymère est constitué d'un matériau polymère choisi parmi le polyéthylène, le polypropylène, le chlorure de polyvinyle, le polycarbonate, le SEBS ou le polyuréthane ou leurs mélanges.

5. Procédé selon l'une des revendications 1 à 4, dans lequel le substrat polymère est un dialyseur, un tube, un cathéter, un stent ou un urètre ou au moins forme une partie de ceux-ci.

6. Procédé selon l'une des revendications 1 à 5, dans lequel le composé photo-initiateur non toxique est choisi dans le groupe comprenant les flavines, flavones, flavonoïdes et leurs dérivés et l'amide d'acide nicotinique et ses dérivés et le thioxanthone.

7. Procédé selon la revendication 6, dans lequel le composé initiateur est la riboflavine, la morine, la rutine ou un mélange de celles-ci.

8. Procédé selon la revendication 6, dans lequel le composé initiateur est l'amide d'acide nicotinique.

9. Procédé selon la revendication 6, dans lequel le composé initiateur est le thioxanthone.
